# EUROPEAN PATENT APPLICATION

(11) **EP 1 649 930 A1**
(43) Date of publication of application: **26.04.2006**
(21) Application number: 04105215.0
(22) Date of filing: 21.10.2004
(51) Int. Cl.: B01J 31/18, B01J 31/22, B01J 31/12

(54) **New single catalyst component with metal containing chelate backbone**

(71) Applicant: TOTAL PETROCHEMICALS RESEARCH FELUY, 7181 Seneffe (Feluy) (BE)
(72) Inventor: Gladysz, John, D-91056, Erlangen (DE); Hahn, Christine, D-91056, Erlangen (DE); Tuba, Robert, H-4741, Jankmajtis (HU); Razavi, Abbas, B-7000, Mons (BE)

(57) **Abstract**

The present invention discloses a chelate ligand wherein the backbone contains a metal.

## Description

The present invention relates to the field of catalyst systems based on new single site catalyst components with a metal containing chelate backbone. These comlexes are suitable for the oligomerisation of olefins.

Transition metal complexes with *NO* chelate ligands have been intensively investigated in view of developing homogeneous catalyst systems. Salicylaldimine derivatives have been considered as important ligands in various catalytic systems such as for example ruthenium carbene complexes for alkene metatesis as described for example by Opstal and Verpoort (Opstal T., Verpoort F., in J. Mol. Cat. A: Chem., 2003, 200, 49.) or to form neutral nickel(II) complexes which are highly active alkene polymerisation catalysts as described for example by Ittel et al. (Ittel S.D., Johnson L.K., Brookhart M., in Chem. Rev. 2000, 100, 1169.) and can furthermore function in single-component mode such as described by Younkin et al. (Younkin T.R., Conner E.F., Henderson J.I., Friedrich S.F., Grubbs R.H., Bansleben D.A., in Science, 2000, 287, 460.)

Different features of the chelate ligand have been extensively studied, such as for example by Hicks et al. (Hicks F.A., Jenkins J.C. Brookhart M., in Organometallics 2003, 22, 3533.) who studied the *NO* chelate ring size. The electronic and steric influences of substituents on the chelate backbones have also been investigated: it was found for example by Shim et al. (Shim C.B., Kim Y.H., Lee B.Y., Dong Y., Yun H., in J. Organomet. Chem. 2003, 675, 72) that very bulky substituents at the N-aryl group in salicylaldiminato complexes play a key role in the production of high molecular weight polymers and in catalytic activity.

Very recently, a remarkable electronic fine tuning of the branching and molecular weight by remote substituents at the N-aryl group have been elaborated and described by Zuideveld et al. (Zuideveld M., Wehrmann P., Röhr C., Mecking S. in Angew. Chem. Int. Ed. 2004, 43, 869.).

There is however still a need to understand better the role of the substituents and electronic structure of these complex in order to better taylor the polymers produced with these complexes.

It is an aim of the present invention to prepare chelate complexes with metal containing ligand backbones.

It is another aim of the present invention to provide a method for preparing these chelate complexes.

It is also an aim of the present invention to use these complexes for oligomerising or polymerising alpha-olefins.

Accordingly, the present invention discloses chelate complexes with a metal-containing ligand backbone of general formula I 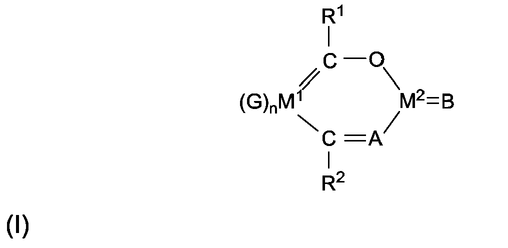 wherein G is a group that inhibits the activity of metal M¹ such as for example an CO group or monodentate or bidentate amine or phosphine or cyclopentadienyl or any similar commonly used ligand;
wherein metal M¹ in the backbone is a metal Group 6-9 of the periodic Table;
wherein metal M² in the chelate complex is a metal Group 10 of the Periodic Table;
wherein R¹, R² are each independently selected from hydrocarbyls having from 1 to 20 carbon atoms;
wherein A is O or N-R^{#}, wherein R^{#} is selected from hydrocarbyls having 4-20 carbon atoms;
wherein B is an allyl or represents an hydrocarbyl group having from 1 to 20 carbon atoms and a pyridine or a similar nitrogen heterocycle, organonitrile or phosphine group; and
wherein n is the valence of M¹ minus 2.

Preferably M¹ is selected from Re or Mn.

Preferably M² is selected from Ni or Pd.

Preferably, G is a CO group.

Preferably, R¹ and R² are each independently alkyl groups having from 1 to 6 carbon atoms or aryl groups having from 6 to 10 carbon atoms, more preferably, they are the same and they are methyl groups.

In a first embodiment, the present invention provides chelate complexes of general formula II 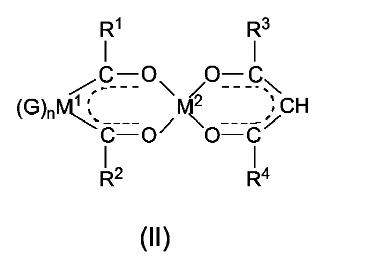 wherein metal M¹, M², n, R¹ and R² are as defined here-above; and
wherein R³ and R⁴ are each independently selected from hydrocarbyls having from 1 to 20 carbon atoms.

Preferably, R³ and R⁴ are each independently alkyl groups having from 1 to 6 carbon atoms or aryl groups having from 6-10 carbon atoms, more preferably, they are the same and they are methyl groups.

In a second embodiment, the present invention discloses chelate complexes of formula III 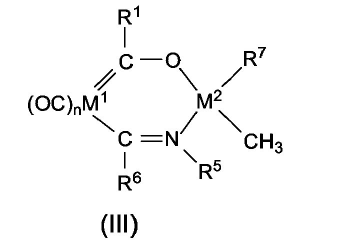 wherein R⁵ is a hydrocarbyl having from 1 to 20 carbon atom, wherein R⁶ is a hydrocarbyl having from 1 to 20 carbon atom or a fully or partially halogenated hydrocarbyl having from 1 to 20 carbon atoms and wherein R⁷ is an amine or phosphine.

Preferably R⁵ is an aryl group, more preferably a substituted aryl group, most preferably, it is a substituted phenyl group.

Preferably R⁶ is a fully haloganated alkyl having from 1 to 6 carbon atoms, more preferably, it is CF₃.

Preferably, R⁷ is pyridine.

In a third embodiment the present invention discloses chelate complexes of formula IV 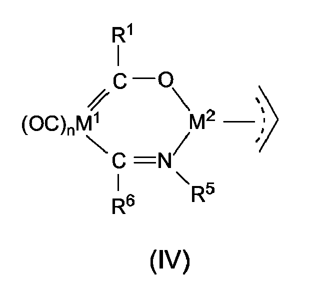 wherein all symbols are as described here-above.

The present invention also discloses a method for preparing the complexes of formula I, II, III and IV.

There are two general methods for preparing complex I starting from the chelate ligand of the general formula V

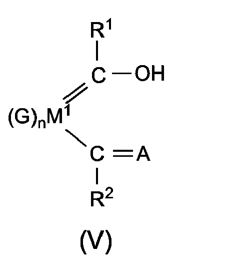

In a first method, the chelate ligand V is reacted:
- first with M³Y wherein M³ can be selected from Li, Na or K, wherein Y is a base and can be selected from R*, OR* or NR*₂, wherein R* can be any hydrocarbyl group,and
- second with LXM²=B, wherein M² and B are as defined here-above, whereinX can be selected from halides or sulfonates and wherein L is preferable a phosphine;
- to form complex I with elimination of HY and M³X.

When B is an allyl, L represents XM²=B and only a half equivalent is applied.

In a second method, the chelate ligand V is reacted with LR^{x}M²=B, wherein R^{x} is a hydrocarbyl and L is any neutral donor ligand like amine or phosphine, with elimination of L and HR^{x}.

The method for preparing the complex of formula II comprises the steps of:
a) providing a complex of general formula VI 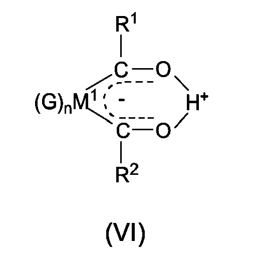
b) reacting the compound of step a) with a metal compound of formula M²(Gr)ᵥ
   wherein Gr is a base that combines with H and v is the valence of metal M², to give a compound of formula II with elimination of HGr
Gr can be selected from R*, OR*, NR*₂, wherein R* can be any hydrocarbyl.

The method for preparing the complex of formula III comprises the steps of:
a) providing a compound of general formula Y[M¹(G)ₘ] wherein Y can be selected from Li, Na, K and wherein m is the valence of M¹ minus 1 and reacting in a solvent, this compound with a compound of general formula 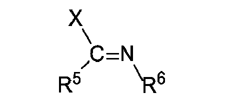 wherein X is a halogen or other leaving group, with liberation of product YX and production of compound VII 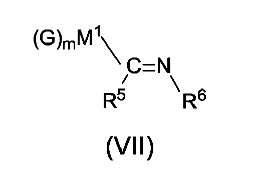
b) reacting compound VII in a solvent with a compound of formula R¹Z wherein Z can be selected from Li, Na or K and wherein R¹ is an alkyl group having from 1 to 10 carbon atoms to provide a compound of formula VIII 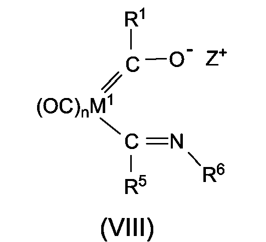
c) reacting compound VIII with an acid HX to provide compound of formula IX with release of a salt of ZX 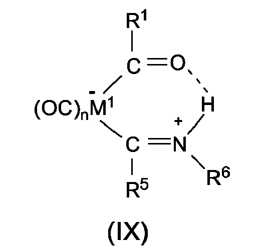
d) reacting compound IX with a diamine compound of metal M² of general formula X 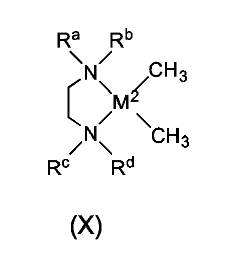 wherein R^{a}, R^{b}, R^{c} and R^{d} are not especially limited and can each be independently selected from a hydrocarbyl having from 1 to 20 carbon atoms, said reaction being carried out in a solvent and optionally with a stabilising agent
e) retrieving compound of formula III with release of methane and of the relevant diamine.

Typically, the solvent can be selected typically from diethylether (Et₂O) or tetrahydrofuran (THF).

Preferably all R^{a}, R^{b}, R^{c} and R^{d} are the same and are methyl groups, in which case the relevant diamine released is tetramethyldiamine.

The stabilising agent is preferably present and it is selected from CH₃CN, or other organonitriles, or pyridine or similar nitrogen heterocycles. More preferably, it is pyridine.

The complex of formula IV can be prepared by different routes and the method of preparation is selected according to the nature of metal M².

The method for preparing the complex of formula IV wherein M² is Ni comprises the steps a) through c) used in the preparation of the metal compound of formula III followed by:
d) reacting in a solvent the complex of formula IX of step c)
   - with compound R⁸Z wherein R⁸ is a hydrocarbyl having from 1 to 20 carbon atoms; and
   - with a nickel compound of formula [{Ni(allyl)X}₂| wherein the allyl is unsubstituted or substituted and wherein X is a halogen
   - at a temperature of less than 0 °C and during a period of time of from 30 minutes to 2 hours
e) retrieving a nickel complex of general formula IV with release of ZX and an alkane R⁸H.

Preferably, Z is lithium, X is chlorine and R⁸ is butyl.

Preferably, the temperature is of less than -10 °C, more preferably of less than -20 °C and most preferably of about -78 °C.

Preferably, the reaction is carried out for a period of time of about one hour.

The method for preparing the complex of formula IV wherein M² is Pd comprises the steps a) through b) used in the preparation of the metal compound of formula III followed by:
b) reacting in a solvent the complex of formula VIII of step b) with a palladium compound of formula [{Pd(allyl)X}₂| wherein the allyl is unsubstituted or substituted and wherein X is a halogen at a temperature of from 0 °C to room temperature (about 25 °C) and during a period of time of from 30 minutes to 2 hours;
c) retrieving the complex of formula IV with release of ZX.

In further embodiments according to the present invention, one or more CO groups appearing in the backbone of the chelate ligands can be replaced by amines such as for example pyridine, dipyridine, phenanthroline.

The complexes according to the present invention can be used to oligomerise alpha-olefins.

Preferably an activating agent is used in order to improve the activity of the system.

The present invention discloses an active oligomerisation catalyst system comprising:
- any one of the metal complexes described here-above;
- optionally an activating agent; and
- optionally a support.

The activating agent can be an aluminium alkyl represented by formula AIR^{$}ₙX₃₋ₙ wherein R^{$} is an alkyl having from 1 to 20 carbon atoms and X is a halogen. The preferred alkylating agents are triisiobutyl aluminium (TIBAL) or triethyl aluminium (TEAL).

Alternatively, an alumoxane can be used as activating agent. The alumoxanes are well known and preferably comprise oligomeric linear and/or cyclic alkyl alumoxanes represented by the formula: 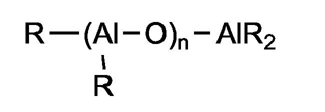 for oligomeric, linear alumoxanes and 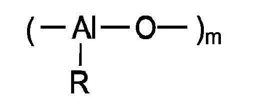 for oligomeric, cyclic alumoxane,
wherein n is 1-40, preferably 10-20, m is 3-40, preferably 3-20 and R is a C₁-C₈ alkyl group and preferably methyl.

Suitable boron-containing activating agents may comprise a triphenylcarbenium boronate such as tetrakis-pentafluorophenyl-borato-triphenylcarbenium as described in EP-A-0427696, or those of the general formula [L'-H] + [B Ar₁ Ar₂ X₃ X₄]- as described in EP-A-0277004 (page 6, line 30 to page 7, line 7).

Optionally, the catalyst component can be supported on a support. Preferred supports include a porous solid support such as talc, inorganic oxides and resinous support materials such as polyolefin. Preferably, the support material is an inorganic oxide in its finely divided form.

Suitable inorganic oxide materials are well known in the art. Preferably, the support is a silica support having a surface area of from 200-700 m²/g and a pore volume of from 0.5-3 ml/g.

The amount of activating agent and catalyst component usefully employed in the preparation of the solid support catalyst can vary over a wide range and depend upon the nature of the activating agent and of the metal. Typically, the ratio AI/M² varies from 100 to 2000.

The present invention also discloses a method for oligomerising alpha-olefins that comprises the steps of:
a) injecting into the reactor the active, optionally supported, catalyst system described here-above;
b) injecting a monomer and optional comonomer into the reactor;
c) maintaining under oligomerisation conditions;
d) retrieving the oligomer.

The oligomerisation conditions are not particularly limited. The temperature may vary from 20 to 80 °C, preferably from 30 to 40 °C and the pressure can vary from 1 to 10 bars, preferably from 3 to 8 bars. The oligomerisation is typically carried out for at least one hour.

The monomers and comonomers are typically selected from alpha-olefins but they can include styrene and polar monomers such as for example methacrylates and methylvinyl ketones. Preferably they are selected from ethylene and propylene.

### List of figures.

Figure 1 represents reaction scheme 1 used for the preparation of rhena-β-ketoimines complexes 2 to 5.
Figure 2 represents reaction scheme 2 used for the preparation of imidoyl pentacarbonyl rhenium complexzs 6 and 7 and of rhena-β-imine complex 8.
Figure 3 represents reaction scheme 3 used in the preparation of bipyridine rhena-β-diketone (complex 9), pyridine imidoyl rhenium (complex 10) and phenanthroline imidoyl rhenium (complex 11).
Figure 4 represents reaction scheme 4 used in the preparation of rhena-β-diketonato nickel(II) complex (12).
Figure 5 represents reaction scheme 5 used for the preparation of rhena-β-ketoiminato allyl nickel(II) complexes 13 and 14.
Figure 6 represents reaction scheme 6 used for the preparation of rhena-β-ketoiminato methyl nickel(II) complex 15.
Figure 7 represents reaction scheme 7 used for the preparation of rhena-β-ketoiminato allyl palladium(II) complexes 16, 17 and 18.

### Examples.

All operations were carried out under nitrogen atmosphere. THF, diethyl ether, pentane, and toluene were distilled from Na/benzophenone, and CH₂Cl₂ was distilled from CaH. The primary amines and pyridine were dried with KOH and distilled before use. Alkyl lithium reagents, bipyridine, and phenanthroline were received from Fluka, Aldrich and ACROS, Ni(acac)₂ from ABCR, MAO from ATOFINA, [{Pd(allyl)Cl}₂] from Aldrich. Rhena-β-diketone, Trifluoroacetylimidoyl chlorides, [{Ni(allyl)Cl}₂], and [Ni(tmeda)(CH₃)₂] were prepared according to literature procedures, respectively from Lukehart and Zeile (Lukehart C.M., Zeile J.V., in J. Am. Chem. Soc. ,98, 2365, 1976), Tamura et al. (Tamura K., Mizukami H., Maeda K., Watanabe H., Unayama K., in J. Org. Chem., 58, 32, 1993), Taube et al. (Taube R., Böhme P., Gehrke J.-P., in Z. Anorg. Allg. Chem. 578, 89, 1989) and Kaschube et al. (Kaschube W., Pörschke K. R., Wilke G., in J. Organomet. Chem., 355, 525, 1988). NMR spectra were recorded on Bruker 300 and 400 MHz spectrometers, and IR spectra were measured on a ASI React-IR spectrometer. Gas chromatography was conducted on a ThermoQuest Trace GC 2000 instrument.

### Preparation of rhena-β-ketoimines (complexes 2 to 5).

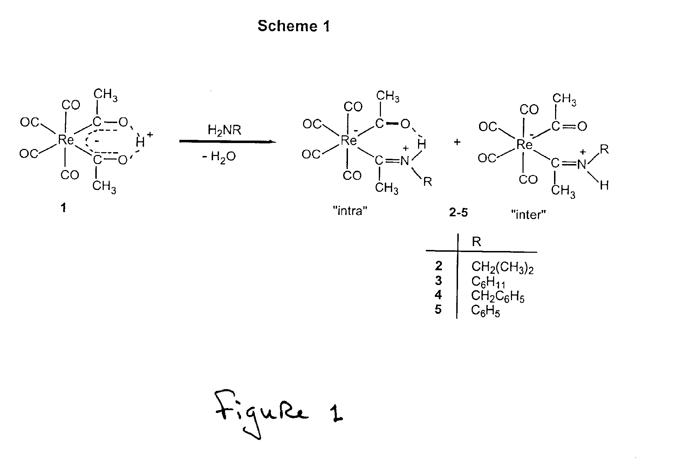

These preparations were performed according to scheme 1 represented in Figure 1 and described by Lukehart and Zeile (Lukehart C.M., Zeile J.V., in J. Am. Soc. 100, 2774, 1978).
Starting complex 1 was

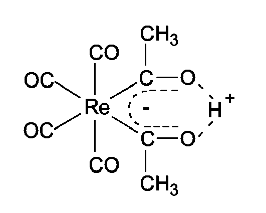

To a solution of 0.5 mmol of starting complex 1 in 5 ml of CH₂Cl₂ , 3.0 mmol of RNH₂ were added, wherein R was respectively selected from CH₂CH(CH₃)₂, or C₆H₁₁, or CH₂C₆H₅ or C₆H₅. The mixture was stirred at room temperature for a period of time depending upon the nature of R. It was respectively of 1 h for R = CH₂CH(CH₃)₂ of 6 h for R = C₆H₁₁, of 9 h for R = CH₂C₆H₅ and of 48 h for R = C₆H₅. The solvent was removed under reduced pressure. The residue was dissolved in pentane and after filtration the solvent was removed under reduced pressure giving a pale yellow oil-solid mixture. In the case of aniline the reaction was performed in Et₃N as solvent and 4 angströms molecular sieves were added. The mixture was stirred very slowly during 2 days. The solvent was removed under reduced pressure. The residue was extracted with about 10 ml of pentane and after removal of the pentane a yellow oil was retrieved as crude product, which was purified by chromatography with silica gel. The yields were in the range of 50 to 60%.

¹H NMR analysis was carried out on all final products and the following results were obtained.
- For R = CH₂CH(CH₃)₂ (complex 2) ¹H NMR (CDCl₃): δ (intra isomer) 1.07 (d, J_{HH} = 6.7 Hz, 6H, CH₃), 2.05 (m, 1 H, CH), 2.50 (s, 3H, CH₃C=O), 2.84 (s, 3H, CH₃C=N), 3.57 (ps t, J_{HH} = 4 Hz, 2H, CH₂), 9.16 (br, 1 H, NH). δ (inter isomer) 1.11 (d, J_{HH} = 6.7 Hz, 6H, CH₃), 2.16 (m, 1 H, CH), 2.58 (s, 3H, CH₃C=O), 2.69 (s, 3H, CH₃C=N), 3.36 (ps t, J_{HH} = 4 Hz, 2H, CH₂), 12.90 (br, 1 H, NH).
- For R= C₆H₁₁, (complex 3) ¹H NMR (CDCl₃): δ (intra isomer) 1.19-1.97 (m, 10 H, C₆H₁₁), 2.48 (s, 3H, CH₃C=O), 2.73 (s, 3H, CH₃C=N), 3.84 (m, 1 H, CH), 9.16 (br, 1 H, NH). δ (inter isomer) 1.19-1.97 (m, 10 H, Cy,), 2.58 (s, 3H, CH₃C=O), 2.68 (s, 3H, CH₃C=N), 2.90 (m, 1 H, CH), 12.80 (br, 1 H, NH).
- For R= CH₂C₆H₅, (complex 4) ¹H NMR (CDCl₃): δ (intra isomer) 2.56 (s, 3H, CH₃C=O), 2.86 (s, 3H, CH₃C=N), 4.90 (d, J_{HH} = 6.0 Hz, 2H, CH₂), 7.26-7.81 (m, 5H, C₆H₅), 9.01 (br, 1 H, NH). δ (inter isomer) 2.61 (s, 3H, CH₃C=O), 2.75 (s, 3H, CH₃C=N), 4.74 (d, J_{HH} = 5.4 Hz, 2H, CH₂), 7.26-7.81 (m, 5H, C₆H₅), 13.41 (br, 1 H, NH).
- For R= C₆H₅, (complex 5) ¹H NMR (CDCl₃): δ (inter isomer) 2.70 (s, 3H, CH₃), 2.77 (s, 3H, CH₃), 7.15-7.28 (m, 2H, Ph), 7.42-7.52 (m, 2H, Ph), 14.89 (br, 1 H, NH).

### Preparation of imidoyl pentacarbonyl rhenium complexes 6 and 7 and of rhena-β-imines (complex 8).

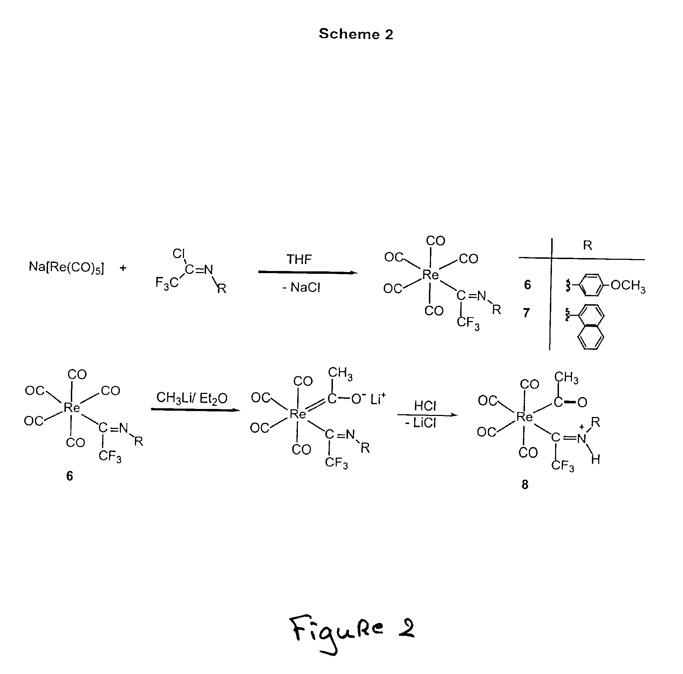

These preparations were performed according to new scheme 2 represented in Figure 2.
A solution of 1.65 mmol of Na[Re(CO₅)] in 30 ml of THF was reacted with 1.63 mmol of F₃CC(Cl)=NR wherein R was respectively selected from p-CH₃OC₆H₄ or naphthyl. During the reaction a white precipitate was formed and the solution turned light yellow. After one day the suspension was filtered off and the solvent of the filtrate was removed under reduced pressure. The yellow residue was dissolved in 100 ml of pentane and the solution was stirred for one hour, the precipitate was filtered off and the volume of the filtrate was reduced under reduced pressure. Light yellow crystals precipitated. The yields were of 95% for both cases.

¹H NMR and infrared (IR) analyses were carried out on all final products and the following results were obtained.
- For R= p-CH₃OC₆H₄, (complex 6) ¹H NMR (CDCl₃): δ 3.69 (s, 3H, OCH₃), 6.40 (d, J_{HH} = 8 Hz, 2H, C₆H₄), 6.78 (d, J_{HH} = 8 Hz, 2H, C₆H₄) and ¹³C NMR (CDCl₃): δ 55.6 (s), 114.9 (s), 119.5 (s), 123.2 (q, J_{CF} = 280 Hz, CF₃), 151.4 (s), 157.5 (s), 179.6 (s), 180.3 (q, J_{CF} = 34 Hz, ReCCF₃), 180.7 (s), and IR (THF, v cm⁻¹): 2140 , 2056, 2011, 1993.
- For R = naphthyl (complex 7); ¹H NMR (C₆D₆): δ 6.40 (d, J_{HH} = 7.3 Hz, 1 H), 7.16 (m, 3H), 7.48 (d, J_{HH} = 8.3 Hz, 1 H), 7.57 (d, J_{HH} = 8.1 Hz, 1 H), 7.73 (d, J_{HH} = 8.1 Hz, 1 H). ¹³C NMR(C₆D₆): δ 112.3 (s), 123.4 (s), 125.0 (s), 125.5 (s), 126.0 (s), 126.1 (s), 126.9 (s), 128.1(s), 134.6 (s), 154.2 (s), 124.2 (q, J_{CF} = 280 Hz, CF₃), 180,0 (q, J_{CF} = 35.3 Hz, ReCCF₃), 179.4 (s), 180.6 (s). IR (THF, ν cm⁻¹):2138,2026,2009.
   640 mg (1.22 mmol) of complex 6 prepared in the preceding step were reacted with 0.83 ml (1.22 mmol) of CH₃Li in 40 ml of diethyl ether at a temperature of -78 °C. After stirring the reaction mixture for two hours, 6 ml (1.22 mmol) of a saturated HCl solution in diethyl ether was added at a temperature of -78°C and kept under stirring for 15 minutes. The mixture was then warmed up to 0°C and was stirred for another hour at 0°C. The solvent was removed under reduced pressure, and the yellow residue was extracted with 120 ml of n-hexane at room temperature. After filtration of the solution and removal of the solvent under reduced pressure 333 mg yellow oil (complex 8) were obtained with a yield of 50%.

NMR and IR analyses were carried out with the following results.

¹H NMR (CDCl₃): δ 2.61 (s, 3H, CH₃), 3.74 (s, 3H, OCH₃), 6.85 (d, J_{HH} = 9.0 Hz, 2H, C₆H₄), 7.09 (d, J_{HH} = 9.0 Hz, 2H, C₆H₄), 16.11 (s, 1 H, NH), ¹³C NMR (CDCl₃): δ 28.69 (s), 54.6 (s), 113.5 (s), 123.2 (s), 128.2 (q, J_{CF} = 280 Hz, CF₃),152.8 (s), 159.0 (s), 186.6 (s), 236.6 (q, ReCCF₃). IR (Et₂O, v cm⁻¹): 2080, 1985, 1955, 1914.

One or more of the CO groups in starting complex 1 or complex 6 can be replaced by nitrogen-containing compounds.

### Preparation of bipyridine rhena-β-diketone (complex 9).

197 mg (0.51 mmol) of starting complex 1 was reacted with 85 mg (0.54 mmol) of bipyridine in 14 ml of toluene at room temperature for a period of time of 3 days. During this period a bright yellow powder precipitated gradually from the reaction solution. The powder was collected by filtration, washed with 5 ml of hexane and dried under vacuum. 70 mg (0.144 mmol) of complex 9 were obtained with a yield of 28%.

NMR and IR analyses were carried out with the following results.

¹H NMR (CDCl₃): δ 1.49 (s, 6H, CH₃), 7.42 (m, 2H, bpy), 7.97 (m, 2H, bpy), 8.05 (m, 2H, bpy), 9.10 (d, J_{HH} = 5 Hz, 2H, bpy), 19.91 (s, 1 H, H). ¹³C NMR (CDCl₃): δ 23.3 (s), 122.6 (s), 126.8 (s), 138.9 (s), 153.3 (s), 154.4 (s), 176.8 (s), 179.6 (s). IR (Et₂O, v cm⁻¹): 2023, 2014, 1911, 1893.

### Preparation of pyridine imidoyl rhenium (complex 10).

100 mg (0.189 mmol) of complex 6 were dissolved in 5 ml of toluene to which were added 15.7 mg (0.198 mmol) of pyridine. The reaction mixture was refluxed overnight while the colour changed from light yellow to cherry red. The toluene was removed under reduced pressure and the remaining reddish oil was washed with 3 ml of n-hexane, and dried under vacuum. 98 mg (0.17 mmol) of complex 10 were obtained with a yield of 91 %.
IR analysis was carried out and gave the following result: IR (CH₂Cl₂, v cm⁻¹): 2086 , 1978, 1939, 1893.

### Preparation of phenanthroline imidoyl rhenium (complex 11).

100 mg (0.189 mmol) of complex 6 was reacted with 36 mg (0.2 mmol) of 1,10-phenanthroline in 5 ml of refluxing toluene. Within 1 day the colour changed from light yellow to dark orange. Upon cooling orange red crystals formed which were filtered off and washed with 3 ml of n-hexane and dried under vaccum. 74 mg (0.11 mmol) of complex 11 were obtained with a yield of 60%.

NMR and IR analyses results were as follows.

¹H NMR (C₆D₆): δ 3.40 (s, 3H, OCH₃), 6.15 (d, J_{HH} = 8.8 Hz, 2H C₆H₄), 6.37 (d, J_{HH} = 8.8 Hz, 2H C₆H₄), 6.41 (m, 2H, phen), 6.80 (s, 2H, phen), 7.07 (m, 2H, phen), 8.55 (d, J_{HH} = 5 Hz, 2H, phen). ¹³C NMR (C₆D₆): δ 55.6 (s), 113.8 (s), 118.9 (s), 124.9 (s), 125.4 (q, J_{CF} = 280 Hz, CF₃), 127.2 (s), 130.2 (s), 136.5 (s), 146.4 (s), 148.4 (s), 154.0 (s), 155.1 (s), 191.2 (s), 198.2 (s), 201.5 (q, J_{CF} = 31 Hz, ReCCF₃) and IR (CH₂Cl₂, v cm⁻¹): 2011, 1893.

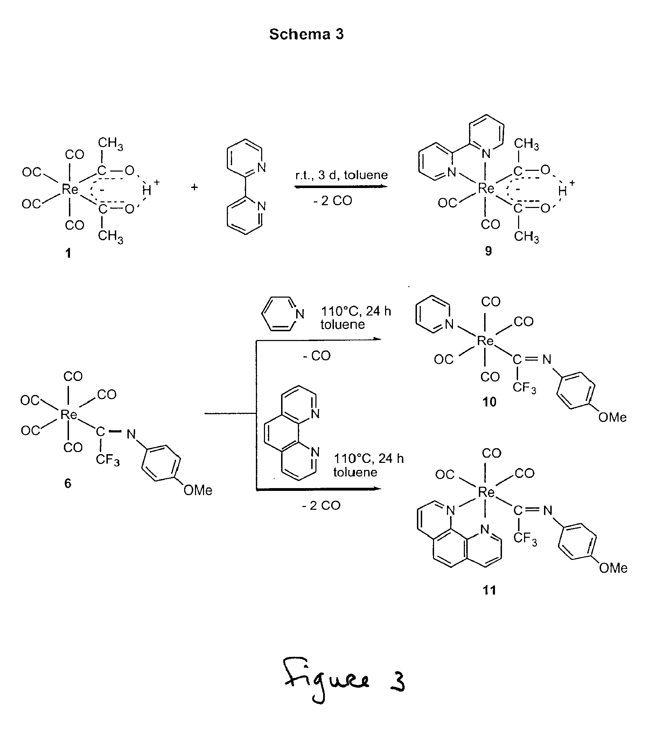

Preparation of complexes 9, 10 and 11 is represented schematically in Figure 3.

### Preparation of rhena-β-diketonato nickel(II) complex (complex 12).

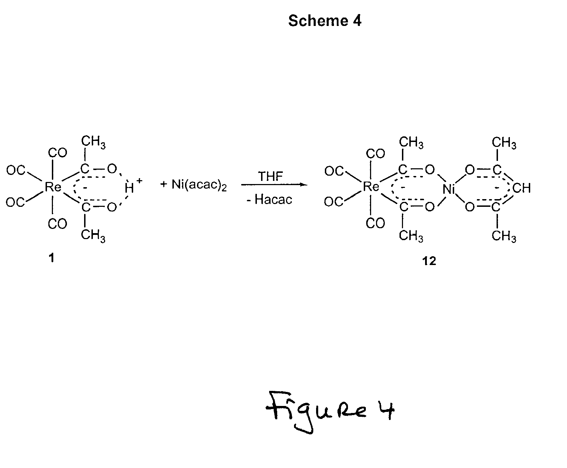

A solution of 99 mg (0.389 mmol) of Ni(acac)₂ in 7 ml of THF was added to a solution of 150 mg (0.389 mmol) of complex 1 in 5 ml of THF. The reaction mixture was stirred for a period of time of 3 hours. The solvent was removed under reduced pressure and the residue was re-crystallized from pentane giving a blue-green crystalline solid (complex 12). This preparation is represented schematically in Figure 4.

NMR and IR analyses were carried out on complex 12 and gave the following results.

¹H NMR (C₆D₆): δ 1.80 (s, 3H, CH₃), 2.40 (s, 3H, CH₃), 5.34 (s, 1 H, CH) and IR (v cm⁻¹): 2081, 2050, 1957, 1922, 1590, 1513, 1447, 1386, 1336, 1262, 1131, 1089, 1019, 926, 779.

### Preparation of rhena-β-ketoiminato allyl nickel(II) complexes 13 and 14.

To a solution of 200 mg (0.454 mmol) of complex 2 in 7 ml of THF 0.283 ml of "BuLi (1.6 M in hexane) were added at a temperature of -78°C. After stirring at -78°C for one hour, a solution of 61 mg (0.454 mmol Ni) of [{Ni(allyl)Cl}₂] in 2 ml of THF was added at the same temperature. The reaction mixture was stirred for another hour at -78°C. Upon removal of the solvent a dark red brown solid was obtained. In the same manner complex 5 was reacted subsequently with ⁿBuLi and [{Ni(allyl)Cl}₂] giving also a dark red brown solid.

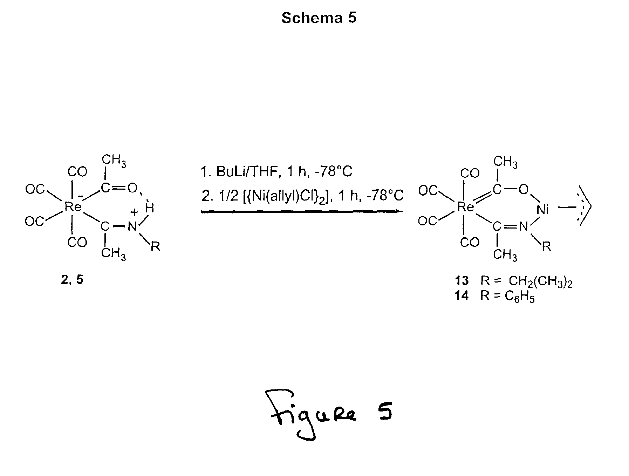

Complexes 13 and 14 are represented schematically in Figure 5 and they were characterized by ¹H NMR in C₆D₆.
For R = CH₂CH(CH₃)₂ (complex 13) ¹H NMR (C₆D₆): δ 0.54 (d, 3H, CH₃), 0.57 (d, 3H, CH₃), 1.38 (s, 3H, CH₃CO), 1.96 (s, 3H, CH₃CN), 3.98 (d, allyl), 4.63 (m, allyl).
For R = C₆H₅ (complex 14) ¹H NMR (C₆D₆): δ 1.27 (s, 3H, CH₃CO), 1.74 (s, 3H, CH₃CN), 4.03 (d, allyl), 4.73 (m, allyl), 6.49 (m, C₆H₅), 6.99 (m, C₆H₅) .

### Preparation of rhena-β-ketoiminato methyl nickel(II) complex 15.

To a solution of 420 mg (0.95 mmol) of complex 2 and 195 mg (0.95 mmol) of [Ni(tmeda)(CH₃)₂] in 20 ml of diethyl ether, 0.77 mml (9.5 mmol) of pyridine were added. After stirring the reaction mixture for 6 hours at room temperature, the dark red brown precipitate was filtered off the crude product and was then washed with pentane and dried in vacuum.

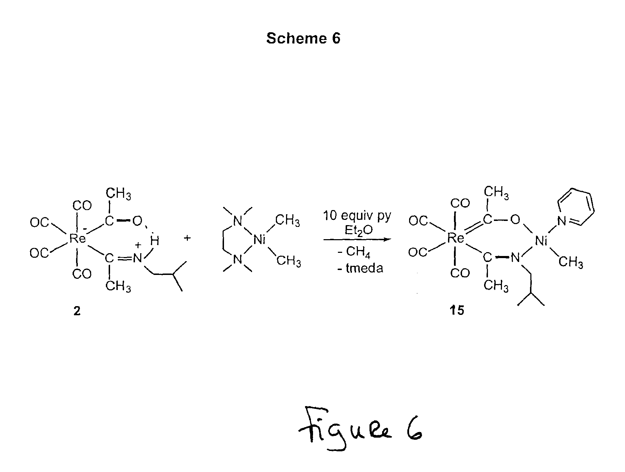

This preparation is represented schematically in Figure 6.

NMR analysis gave the following results: ¹H NMR (THF-d8): δ δ -0.57 (s, 3H, NiCH₃,), 0.84 (m, 6H, CH₂CH(CH₃)₂), 2.17 (s, CH₃C=O) 2.57 (s, CH₃C=N), 7.54 (t, 2H, py), 7.94 (t, 1 H, py), 9.17 (d, 2H, py).

### Preparation of rhena-β-ketoiminato allyl palladium (II) complexes 16, 17 and 18.

107 mg (0.189 mmol) of complex 6 were reacted with 0.128 ml of CH₃Li in 10 ml of THF at a temperature of 0°C. The mixture was kept under stirring for one hour and 35 mg (0.096 mmol) of [{Pd(allyl)Cl}₂] were then added to the reaction solution, and the mixture was stirred for a further period of time of 3 hours. Upon addition of the Pd complex the colour of the reaction solution changed from light yellow to dark green. The solvent was removed under reduced pressure and the dark residue was extracted with 70 ml of n-pentane. The solution was filtered and the solvent was removed from the filtrate under reduced pressure giving a green oil (complex 16).

Using exactly the same procedure, complex 6 was reacted with tBuLi and [{Pd(allyl)Cl}₂] giving also a green oil (complex 17).

Using exactly the same procedure, complex 6 was reacted with naphtylLi and [{Pd(allyl)Cl}₂] giving also a green oil (complex 18).

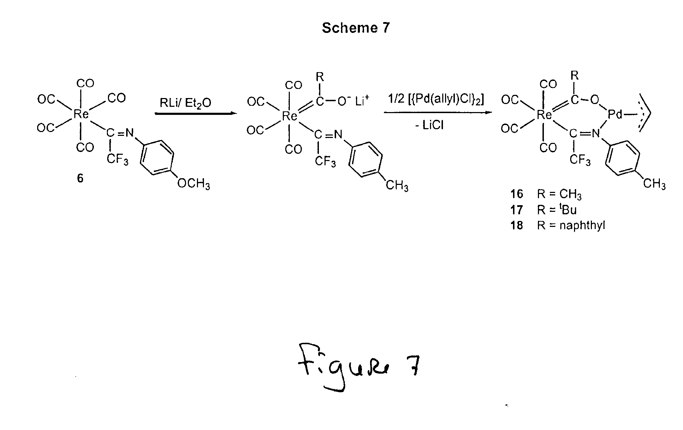

The preparation of complexes 16, 17 and 18 is represented schematically in Figure 7.

NMR and IR analyses were carried out on complexes 16 and 17 with the following results.

74 mg of complex 16 (R = CH₃) were obtained with a yield of 57%.
¹H NMR (C₆D₆): δ 1.34 (s, 3H, CH₃), 3.21 (s, 3H, OCH₃), 2.20 (d, J_{HH} = 13 Hz, 1 H, C₃H₅ (allyl)), 2.24 (d, J_{HH} = 13 Hz, 1 H, C₃H₅ (allyl)), 2.81 (d, J_{HH} = 7 Hz, 1 H, C₃H₅ (allyl)), 3.3 (d, J_{HH} = 8 Hz, 1 H, C₃H₅ (allyl)), 4.53 (m, 1 H, C₃H₅ (allyl)), 6.54 (d, J_{HH} = 8 Hz, 2H, C₆H₄), 6.6 (d, J_{HH} = 8 Hz, 2H, C₆H₄); ¹³C NMR (C₆D₆): δ 30.2 (s), 54.9 (s), 60.0 (s), 65.8 (s), 113.9 (s), 115.5 (s), 120.6 (s), 150.7 (s), 157.3 (s), 187.2 (s), 188.7 (s), 217.9. IR (THF, v cm⁻¹): 2103, 2030, 2009, 1989, 1951.

53 mg of complex 17 (R = C(CH₃)₃) were obtained with a yield of 40%.
¹H NMR (C₆D₆): δ 0.68 (s, 3H, C(CH₃)), 0.87 (s, 3H, C(CH₃)), 1.17 (s, 3H, C(CH₃)); 3.21 (s, 3H, OCH₃), 2.21 (d, J_{HH} = 13 Hz, 1 H, C₃H₅ (allyl)), 2.24 (d, J_{HH} = 12 Hz, 1 H, C₃H₅ (allyl)), 2.82 (d, J_{HH} = 7 Hz, 1 H, C₃H₅ (allyl)), 3.30 (d, J_{HH} = 8 Hz, 1 H, C₃H₅ (allyl)), 4.41 (m, 1 H, C₃H₅ (allyl)), 6.54 (d, J_{HH} = 8 Hz, 2H, C₆H₄), 6.6 (d, J_{HH} = 8 Hz, 2H, C₆H₄); ¹³C NMR (C₆D₆): δ 26.14 (s), 26.72 (s), 28.96 (s), 41.36 (s), 54.85 (s), 60.03 (s), 65.77 (s), 113.86 (s), 115.49 (s), 120.60 (s), 150.69 (s), 157.33 (s), 187.20 (s), 188.72 (s), 191.58 (s), 218.19. IR (THF, ν cm⁻¹ 2103, 2032, 2012, 1989, 1949

Complex 18 (R = naphthyl)
¹H NMR (C₆D₆): δ 3.22 (s, 3H, OCH₃), 2.15 (d, J_{HH} = 13 Hz, 1 H, C₃H₅ (allyl)), 2.35 (d, J_{HH} = 12 Hz, 1 H, C₃H₅ (allyl)), 3.00 (d, J_{HH} = 7 Hz, 1 H, C₃H₅ (allyl)), 3.3 (d, J_{HH} = 7 Hz, 1 H, C₃H₅ (allyl)), 4.52 (m, 1 H, C₃H₅ (allyl)), 6.55 (d, J_{HH} = 9 Hz, 2H, C₆H₄), 6.61 (d, J_{HH} = 6 Hz, 2H, C₆H₄), 7.23-7.25 (m, 4H, naphthyl), 7.60-7.62 (m, 3H, naphthyl); ¹³C NMR (C₆D₆): δ 54.94 (s), 58.9 (s), 68.48 (s), 113.84 (s), 115.23 (s), 120.56 (s), 126.20 (s), 126.60 (s), 126.77 (s), 128,84 (s), 129.68 (s), 130.56 (s), 132.63 (s), 134.34 (s), 150.88 (s), 157.33 (s), 184.94 (s), 186.07 (s), 187.80 (s), 216.71 (q, *J*_{*C-C-F*} = 31 Hz, ReCCF₃). IR (THF, ν cm⁻¹): 2099, 2005, 1993, 1949

### Oligomerisation of ethylene.

A Fischer-Porter-Bottle equipped with a stirring bar was charged with 20 ml of toluene and 1000 equiv of MAO (30% in toluene) and the solution was saturated with ethylene (2 x 5 bar ethylene). In a Schlenk flask 0.020 mmol of respectively complexes 1, 2, 3, and 5 were dissolved in 1 ml of THF. At a temperature of -10°C 1 equiv of ⁿBuLi was added to the solution. After stirring for a period of time of 30 min at the same temperature of -10°C, a solution of 0.010 mmol of [{Ni(allyl)Cl}₂] (= 0.020 mmol Ni) in 1 ml of THF were added. After stirring at -10°C for a period of time of 5 to10 min, the solvent was removed under reduced pressure giving a dark brown solid, which was dissolved in toluene and injected into the prepared Fischer-Porter-Bottle under stirring and under nitrogen flow. The temperature was regulated at 30 °C (± 3°C) and under an ethylene pressure of 6 bar. During the reaction, dark brown flakes precipitated for all 4 complexes. The reaction solution was analysed by ¹H NMR spectroscopy and following the work up described by Faissner and Huttner (Faissner R., Huttner G., in Eur. J. Inorg. Chem. 2239, 2003) GC samples were prepared. The work up with MeOH/HCl did not give solid polymer.

¹H NMR results:
Oligomerisation products with complex 1 ¹H NMR (CDCl₃): δ 0.92 (t, J_{HH} = 7 Hz, CH₃), 1.06 (t, J_{HH} = 8 Hz, CH₃), 1.68 (m, aliphatic H), 2.12 (m, aliphatic H), 4.98 (d, J_{HH} = 10 Hz, vinylic =CH₂), 5.05 (d, J_{HH} = 17 Hz, vinylic =CH₂), 5.49 (m, olefinic =CH), 5.94 (m , vinylic =CH).
Oligomerisation products with complex 3 ¹H NMR (400 MHz, CDCl₃): δ 0.92 (t, J_{HH} = 7 Hz, CH₃), 1.00-1.38 (several overlapping signals), 1.67 (m, aliphatic H), 2.09 (m, aliphatic H), 4.97 (d, J_{HH} = 8.8 Hz, vinylic =CH₂), 5.04 (d, J_{HH} = 17 Hz, vinylic =CH₂), 5.47 (m, olefinic =CH), 5.92 (m , vinylic =CH).
Oligomerisation products with complex 5 ¹H NMR (400 MHz, CDCl₃): δ 0.92-2.18 (a number of overlapping signals, aliphatic H), 4.98 (d, J_{HH} = 10 Hz, vinylic =CH₂), 5.05 (d, J_{HH} = 17 Hz, vinylic =CH₂), 5.28 (m, olefinic =CH), 5.49 (m, olefinic =CH), 5.94 (m, vinylic =CH).

## Claims

1. A chelate ligand with a metal-containing backbone of general formula I 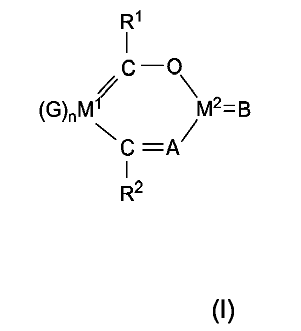 wherein G is a group that inhibits the activity of metal M¹ such as for example an CO group or monodentate or bidentate amine or phosphine or cyclopentadienyl or any similar commonly used ligand;
wherein metal M¹ in the backbone is a metal Group 6-9 of the periodic Table;
wherein metal M² in the chelate ligand is a metal Group10(-4?) (note: typing error or intended?) of the Periodic Table; wherein R¹ and R² are each independently selected from hydrocarbyls having from 1 to 20 carbon atoms;
wherein A is O or NR^{#}, wherein R^{#} is selected from hydrocarbyls having 4-20 carbon atoms;
wherein B is an allyl or represents an hydrocarbyl group having from 1 to 20 carbon atoms and a pyridine group; and
wherein n is the valence of M¹ minus 2.

2. The chelate ligand of claim 1 wherein M¹ is Re or Mn.

3. The chelate ligand of claim 1 or claim 2 wherein M² is Ni or Pd.

4. The chelate ligand of any one of claims 1 to 3 wherein R¹ and R² are each independently alkyl groups having from 1 to 6 carbon atoms or aryl groups having from 6-10 carbon atoms.

5. A method for preparing the chelate ligand of any one of claims 1 to 4 that comprises the steps of
a) providing a chelate ligand of general formula V 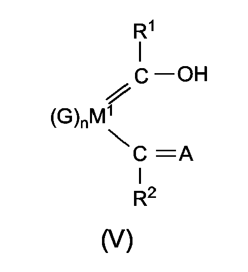
b) reacting compound V with M³Y wherein M³ can be selected from Li, Na or K, wherein Y is a base and can be selected from R*, OR* or NR*₂, wherein R* can be any hydrocarbyl group,and
c) reacting reaction product of step b) with LXM²=B, wherein M² and B are as defined here-above, wherein X can be selected from halides or sulfonates and wherein L is preferable a phosphine;
d) retrieving complex I with elimination of HY and M³X.

6. A method for preparing the chelate ligand of any one of claims 1 to 4 that comprises the steps of
a) providing a chelate ligand of general formula V 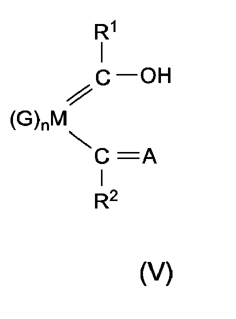
b) reacting compound V with LR^{x}M²=B, wherein R^{x} is a hydrocarbyl and L is any neutral donor ligand like amine or phosphine;
c) retrieving complex I with elimination of HR^{x} and L.

7. An active catalyst system that comprises
a) the chelate ligand of any one of claims 1 to 4;
b) optionally an activating agent;
c) optionally a support.

8. The active catalyst system of claim 7 wherein the activating agent is an alumoxane.

9. A method for oligomerising alpha-olefins that comprises the steps of:
a) injecting into the reactor the active catalyst system of claim 7 or claim 8;
b) injecting the monomer and optional comonomer into the reactor;
c) maintaining under oligomerisation conditions;
d) retrieving an oligomer.

10. The method of claim 9 wherein the oligomerisation is carried out at a temperature of from 20 to 80 °C and a pressure of from 3 to 8 bars.

11. The method of claim 9 or claim 10 wherein the monomer is ethylene or propylene.
